# EUROPEAN PATENT APPLICATION

(11) **EP 4 062 966 A1**
(43) Date of publication of application: **28.09.2022**
(21) Application number: 21166559.1
(22) Date of filing: 01.04.2021
(51) Int. Cl.: A61N 1/32, A61N 1/04

(54) **MULTI-FUNCTIONAL INTEGRATED SKIN BEAUTY DEVICE**

(30) Priority: 25.03.2021 KR 20210039047
(71) Applicant: Soovon Co., Ltd., Incheon 21315 (KR)
(72) Inventor: Oh, Kyung Hee, 10388 Gyeonggi-do (KR)
(74) Representative: Walaski, Jan Filip

(57) **Abstract**

A multi-functional integrated skin beauty device according to the present invention is formed by comprising a mist treatment part generating sterilization water by electrolyzing water in a water tank and spraying the generated sterilization water in a mist shape, a temperature stimulation treatment part applying a temperature stimulation to skin, an EP treatment part applying an electric signal to an EP electrode; and a light treatment part outputting a light for skin care. A housing forming an exterior appearance of the multi-functional integrated skin beauty device includes a pillar-type handle part graspable by a user with one hand, and a first head part and a second head part protruded from an upper end of the handle part toward both lateral surfaces. The multi-functional integrated skin beauty device can care the skin more comfortably and more efficiently because of using not only the hydrogen sterilization water spraying/atomizing function but also the temperature stimulation function, the electroporation function, the light stimulation function, the massage function, and the oil/water measurement function using a single portable device. The multi-functional integrated skin beauty device can maximize a mist spraying efficiency by optimizing the structure of a water tank, increase a user satisfaction by realizing various visual effects and further enhance the safety of the water-using device through the water drainage function of cradle part.

## Description

### [TECHNICAL FIELD]

The teachings in accordance with exemplary and non-limiting embodiments of this invention relate generally to a portable beauty device, and more particularly, to a multi-functional integrated skin beauty device conveniently useable by being effectively integrated with multiple functions including a spraying/atomizing function of hydrogen sterilization water in mist by generating the hydrogen sterilization water through electrolysis, a temperature stimulation function for skin care, an electroporation function, an optical irradiation function, a massage function, and an oil/water measurement function.

### [BACKGROUND OF THE INVENTION]

Recently, women as well as men are particularly concerned about their beauty in order to preserve a beautiful image and appearance. In general, various cosmetics are widely used for beauty treatment such as moisturizing, nutrition supply, collagen that helps activate the skin function, nutritional fluid and essence.

Furthermore, mist suppliers are being used for skin care, where the mist suppliers spout mist of fine particulate state.

The mist supplier may be used for various purposes including, but not limited to, moisturizing, whitening effect, nourishment and the like, and in particular, may be used for multipurpose of skin cleanness.

The laid-open patent No.:10-2020-0086433 discloses a portable mist device that generates hydrogen sterilization water using electrolysis, and spouts the generated hydrogen sterilization water in mist shape through ultrasonic oscillator to thereby be conducive to skin care.

Meantime, skin care may require various elements including not only mist but also temperature stimulation, EP (Electropolation) stimulation, optical stimulation and physical massage through vibration.

For example, the Korean registered patent No.:10-1551923 discloses a hydrogen cosmetics penetration supply device using an electroporation and the Korean registered patent No.:10-2030528 discloses a skin care device generating plasma and electroporation operation to skin. In addition, the temperature stimulation including cold stimulation and warm stimulation adequately applied to skin may provide a desirable beneficial effect to skin care.

However, the disadvantage is that the aforementioned conventional skin care functions cannot be effectively fused together with the hydrogen sterilization water mist function capable of performing to maintain a clean skin.

When the hydrogen sterilization water mist function, which is the most fundamental base for skin care, is not efficiently fused with various functions used for skin care including an EP stimulation, an optical stimulation, and a temperature stimulation, users are forced to use and prepare two or more devices individually to thereby increase an economic burden and to make it difficult to maintain/repair the devices, whereby the skin care may become discomfortable.

### [PriorTechnical Documents]

(1) The Korean Laid-Open Patent No.:10-2020-0086433 (Laid-open on July 17, 2020)
(2) The Korean Registered Patent No.:10-1551923 (Published Date: Sep. 10, 2015)
(3) The Korean Registered Patent No.:10-2030528 (Published Date: Nov. 08, 2019)

### [DETAILED DESCRIPTION OF THE INVENTION]

### [TECHNICAL SUBJECT]

The present invention is devised to solve the aforementioned disadvantages/problems and it is an object of the present invention to provide a multi-functional integrated skin beauty device configured to be realized on a portable device and further improved in use convenience, which is fundamentally equipped with a spraying function of sterilization water in mist using electrolysis, and along with a temperature stimulation function, an electroporation function, an optical stimulation function, a massage function, and an oil/water measurement function.

### [TECHNICAL SOLUTION]

In one general aspect of the present invention, there is provided a multi-functional integrated skin beauty device, the device comprising:
a mist treatment part generating sterilization water by electrolyzing water in a water tank and spraying the generated sterilization water in a mist shape;
a temperature stimulation treatment part applying a temperature stimulation to skin using an thermoelement;
a power supply part supplying an electric power;
an input part receiving an operation command; and
a controller controlling the mist treatment part and the temperature stimulation treatment part in response to the operation command inputted through the input part.

Preferably, but not necessarily, a housing forming an exterior appearance of the multi-functional integrated skin beauty device may include:
a pillar-type handle part graspable by a user with one hand; and
a first head part protruded from an upper end of the handle part to a lateral surface and a second head part protruded to an opposite lateral surface.

Preferably, but not necessarily, the first head part may be disposed with a raw water inlet to allow raw water to be poured into a water tank of the mist treatment part and the second head part may be disposed with a skin contact member to allow applying a temperature stimulation by being contacted with skin.

Preferably, but not necessarily, the multi-functional integrated skin beauty device may further comprise:
an E P treatment part applying an electric signal to an E P electrode; and
a light treatment part outputting a skin care light.

Preferably, but not necessarily, the second head part may be disposed with a transparent window formed at a surrounding of the skin contact member to output a light for skin care.

Preferably, but not necessarily, the skin contact member and an outlying electrode member formed along a surrounding of the transparent window may form the E P electrode.

Preferably, but not necessarily, an uppermost end of the second head part may be disposed higher than the first head part.

Preferably, but not necessarily, the mist treatment part may include:
an electrode module disposed inside of the water tank to transform the raw water to sterilization water by electrolyzing the raw water;
an ultrasonic oscillator generating a fine particulate type mist by applying a vibration to the sterilization water generated by the electrode module; and
a mist outlet to allow spouting the mist generated by the ultrasonic oscillator therethrough.

Preferably, but not necessarily, the water tank may be formed of an approximately '¬' shape across an upper space of the handle part from the raw water inlet.

Preferably, but not necessarily, at least a portion of the electrode module may be so disposed at a place as to be visible through the raw water inlet.

Preferably, but not necessarily, the mist outlet may be disposed at an upper end of the handle part toward a direction formed with the raw water inlet, and the ultrasonic oscillator may be disposed at a surrounding of the mist outlet. At this time, a floor position of the water tank may be so disposed as to be adjacent to a lowermost position of the ultrasonic oscillator.

Preferably, but not necessarily, the multi-functional integrated skin beauty device may further comprise:
an inlet cap configured to open/close the raw water inlet;
a visual effect plate configured to display a visual effect by being so disposed at an inside of the water tank as to allow being visible from the raw water inlet; and
an illumination part configured to shed an illumination light into an inside of the water tank from under the floor of the water tank.

Preferably, but not necessarily, at least a portion of the water tank may be so disposed with a transparent or translucent material as to allow the illumination light coming off from the illumination part to pass therethrough.

Preferably, but not necessarily, at least a portion of the inlet cap may be so disposed as to allow an interior of the water tank to look enlarged.

Preferably, but not necessarily, the multi-functional integrated skin beauty device may further comprise a cradle part so disposed with a battery charging terminal as to allow a lower end of the handle part to rest thereon.

Preferably, but not necessarily, the cradle part may be disposed at a floor with a water drain hole to allow the water flowing down through the handle part to be drained out.

Preferably, but not necessarily, a portion formed with the water drain hole may be formed lower than a portion disposed with the battery charging terminal.

Preferably, but not necessarily, the thermoelement forming the temperature stimulation treatment part may be formed by including a peltier device. At this time, one surface of the peltier device may be so disposed as to thermally contact the water stored in the water tank of the mist treatment part.

Preferably, but not necessarily, the thermal contact may be realized through a first heat dissipation part of first material directly contacting the water stored in the water tank of the mist treatment part and a second heat dissipation part of second material interposed between one surface of the peltier device and the first heat dissipation part.

Preferably, but not necessarily, the first material may be formed with a material less contaminated than the second material by the sterilization water generated through the electrolysis. For example, the first material may include an SU S material and the second material may include an aluminum material.

Preferably, but not necessarily, the second heat dissipation part may be so disposed as to accommodate a cooling liquid at an inside thereof.

Preferably, but not necessarily, the second heat dissipation part may be so disposed as to tight-seal the cooling liquid by closing a lid after the cooling liquid is poured into an inner space thereof.

Preferably, but not necessarily, the thermal contact between the first heat dissipation part and the second heat dissipation part may be realized at an area where the water tank is curbed.

### [ADVANTAGEOUS EFFECTS]

According to the present invention, a user can care skin more simply and more efficiently because the user can utilize not only the hydrogen sterilization water spraying/atomizing function but also the temperature stimulation function, the electroporation function, the light stimulation function, the massage function, and the oil/water measurement function using a single portable device.

Particularly, when the temperature stimulation function, the electroporation function, the light stimulation function, the massage function, and the oil/water measurement function are intensively and collectively performed on one surface contacted by the skin, the skin care can be more effectively realized.

Furthermore, the multi-functional integrated skin beauty device can maximize a mist spraying efficiency by optimizing the structure of a water tank, increase a user satisfaction by realizing various visual effects and further enhance the safety of the water-using device through the water drainage function of cradle part.

### [BRIEF DESCRIPTION OF THE DRAWINGS]

FIG.1 is a functional block diagram illustrating a multi-functional integrated skin beauty device according to an exemplary embodiment of the present invention.
FIG. 2 is an example explaining that a temperature stimulation function, an electroporation function and a light stimulation function are intensively and collectively realized on one surface contacted by the skin.
FIGS 3, 4 and 5 are examples of a housing portably forming a multi-functional integrated skin beauty device according to the present invention.
FIG. 6 is an example illustrating a mist treatment part and a temperature stimulation part each disposed on both sides of a head part of a housing.
FIG. 7 illustrates a mist treatment part according to a detailed exemplary embodiment of the present invention.
FIG. 8 illustrates an upper cross-sectional surface of a housing according to an exemplary embodiment of the present invention.
FIG. 9 illustrates arrangement of skin contact member, transparent window and electroporation electrode according to a detailed exemplary embodiment of the present invention.
FIG. 10 illustrates a temperature stimulation treatment part according to a detailed exemplary embodiment of the present invention.
FIG. 11 illustrates a first heat dissipation part directly contacting sterilization water inside a water tank according to an exemplary embodiment of the present invention.
FIG. 12 illustrates a structure of second heat dissipation part according to a detailed exemplary embodiment of the present invention.
FIG. 13 illustrates an inner appearance of a water tank and illumination effect by allowing an inlet cap to be transparently formed according to a detailed exemplary embodiment of the present invention.
FIG. 14 illustrates a cradle part according to an exemplary embodiment of the present invention.
FIG. 15 illustrates an example of a cross-section of a cradle part.

The present invention may be applied with various changes and have several exemplary embodiments, where particular exemplary embodiments will be exemplified in the drawings and described in detail through the detailed description of the present invention. However, it should be understood that the present invention is not limited to particular embodiments, but encompasses all changes, modifications, equivalents and substitutes included within the ideas and technical scopes of the present invention.

In describing the present invention, detailed descriptions of well-known technologies are omitted for brevity and clarity so as not to obscure the description of the present invention with unnecessary detail.

The terminology used herein is for the purpose of describing exemplary embodiments only and is not intended to be limiting. As used herein, the singular forms may be intended to include the plural forms as well, unless the context clearly indicates otherwise.

It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

It will be understood that, although the terms first, second, etc. may be used herein to describe various elements, these elements should not be limited by these terms. These terms are only used to distinguish one element from another.

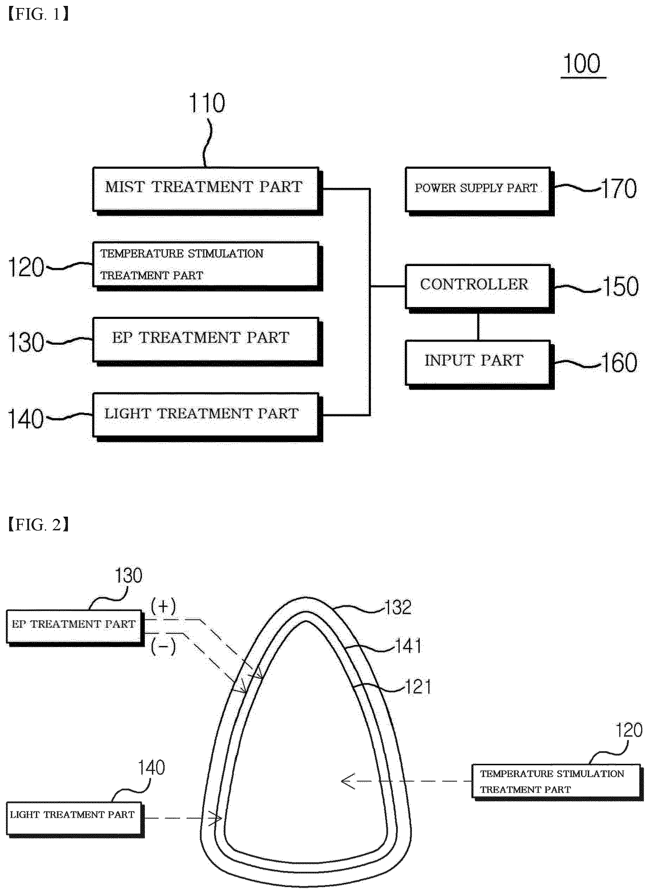

FIG. 1 is a functional block diagram illustrating a multi-functional integrated skin beauty device (100) according to an exemplary embodiment of the present invention, where the device (100) may be formed by including a mist treatment part (110) generating a sterilization water by electrolyzing the water inside a water tank and spraying the generated sterilization water in a mist shape, and a temperature stimulation treatment part (120) applying a temperature stimulation to a user skin by using a thermoelement, and the device (100) may be formed by further including an EP treatment part (130) applying an electric signal to an EP electrode, and a light (optical) treatment part (140) outputting a skin care light capable of being used for skin care. Furthermore, the device (100) may further include a vibration part providing a physical massage and an oil/water measurement part.

Furthermore, the multi-functional integrated skin beauty device (100) may be formed by including an input part (160) receiving an operation command from a user, and a controller (150) controlling the overall operations of the multi-functional integrated skin beauty device (100) including the mist treatment part (110), the temperature stimulation treatment part (120), the EP treatment part (130) and the light treatment part (140) in response to the operation command inputted through the input part (160), and a power supply part (170) supplying an electric power to the device (100).

The input part (160) may receive various operation commands on the multi-functional integrated skin beauty device (100) from a user. The operation commands received from the input part (160) and input methods may be variably configured, and the present invention is not particularly limited thereto.

For example, the input part (160) may include various switches and buttons related to a variety of functions, including but not limited to, power ON/OFF of the multi-functional integrated skin beauty device (100), mist function ON/OFF, temperature stimulation function ON/OFF, EP function ON/OFF, light stimulation ON/OFF, light type set-up function, cold stimulation set-up, warm stimulation set-up, temperature set-up, massage intensity control function, oil/water measurement and contgrol and the like.

The EP treatment part (130) may increase the penetrability of skin cell membrane by applying an electric signal to an electroporation electrode to thereby add an electric field to the skin cell membrane of a user.

The voltages, waveforms and frequencies of the electric signal applied to the electroporation electrode by the EP treatment part (130) may be variably configured according to the need, and the shape, structure and placement of the electroporation electrode may be also variably formed.

The light treatment part (140) may output one or more types of skin-care lights useable for skin care, and may, simultaneously or selectively, output lights of various wavelength ranges (e.g.: 400∼470nm, 570∼590nm, 630∼700nm, 800∼1,200nm, etc.).

The light treatment part (140) may output skin-care lights through an LED (Light-Emitting Diode), and the wavelength, intensity and pattern of lights may be variably organized.

The lights outputted from the light treatment part (140) may show various skin-care effects, including but not limited to, antioxidation, whitening, skin tone-up, wrinkle recovery, sensitive skin soothing, acne improvement, blemishes/pigmentation control, skin soothing, strengthening skin elasticity, improving blood circulation, pore contraction, inhibition of aging, skin regeneration and the like, depending on characteristics thereof.

The temperature stimulation structure for temperature stimulation, the electroporation electrode structure for electroporation stimulation and light output structure for light stimulation and the like may be variably organized.

Particularly, the multi-functional integrated skin beauty device (100) according to an exemplary embodiment of the present invention may be so configured as to be intensively provided on one surface contacted by a user skin with temperature stimulation function, electroporation function and light stimulation function, whereby the skin care effect can be more efficiently improved.

Referring to FIG. 2, the temperature stimulation treatment part (120) may perform the role of applying a temperature stimulation to a user skin by using a thermoelement, and may be formed by including a skin contact member (121) which is an element heated or cooled by being directly contacted to a user skin.

The skin contact member (121) may be disposed on one surface contacted by a user skin at a housing of the multi-functional integrated skin beauty device (100).

Furthermore, a transparent window (141) may be disposed along a surrounding of the skin contact member (121) to allow a light outputted from the light treatment part (140) to be emitted, and an outlying electrode member (132) may be disposed along an outer surrounding of the transparent window (141).

At this time, the skin contact member (121) and the outlying electrode member (132) may be formed with an electrically conductive material, and the skin contact member (121) and the outlying electrode member (132) may form the electroporation electrode.

That is, the skin contact member (121) may be a positive pole (+) or negative pole (-) electrode for electroporation stimulation in addition to the role of applying a temperature stimulation to the skin, and the outlying electrode member (132) may be an electrode having an opposite polarity (positive pole or negative pole) of the skin contact member (121).

Although FIG. 2 has illustrated examples in which each of the skin contact member (121), the transparent window (141) and the outlying electrode member (132) takes approximately a triangular shape, it should be apparent that material, shape and placement or arrangement of the skin contact member (121), the transparent window (141) and the outlying electrode member (132) may be variably organized, and the present invention is not limited thereto. For example, it should be apparent that the skin contact member (121), the transparent window (141) and the outlying electrode member (132) may take an arbitrary shape including a round shape, a polygonal shape including a square shape or an oval shape, and each may not take the same shape.

As one of the embodying methods to allow each function to be intensively and integrally provided on one surface, and each function to cause an upward effect on the other, the transparent window (141) may be formed in a ring shape encompassing an outlying area of the skin contact member (121). Furthermore, the outlying electrode member (132) formed along an outer surrounding of the transparent window (141) may be formed in a ring shape encompassing the transparent window (141).

Although a housing forming an outer appearance of the multi-functional integrated skin beauty device (100) may be variably formed as diverse as possible, it is preferable that the housing be formed with a portable shape to allow a user to use by grasping with one hand.

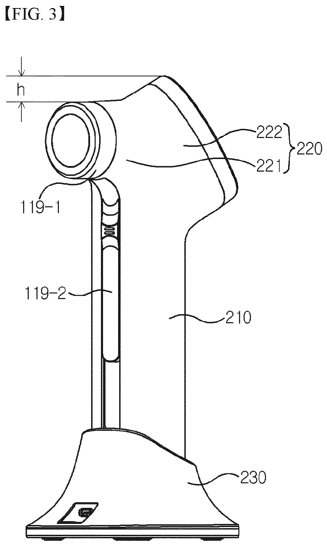

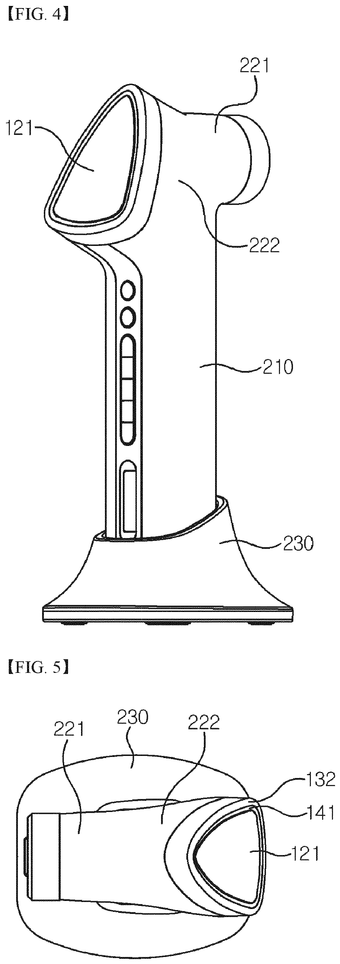

FIGS. 3, 4 and 5 illustrate an example of a housing portably forming the multi-functional integrated skin beauty device (100), where FIG. 3 is a perspective view seen from a direction from which mist is spouted, FIG. 4 is a perspective view seen from a direction where the skin contact member (121) contacts a user skin in order to provide a temperature stimulation, and FIG. 5 is a view seen from a top of a housing.

The housing may be formed by including a pillar-shaped handle part (210) to allow a user to grasp with one hand, and a head part (220) protruded from an upper end of the handle part (210) toward both lateral surfaces thereof. At this time, the head part (220) may include a first head part (221) protruded from an upper end of the handle part (210) to one lateral surface and a second head part (222) protruded toward an opposite lateral surface thereof.

The position (height) and angle protruded by the first head part (221) and the second head part (222) from the handle part (210) may be variably implemented. Furthermore, the shape of the first head part (221) and the second head part (222) may be also variably implemented.

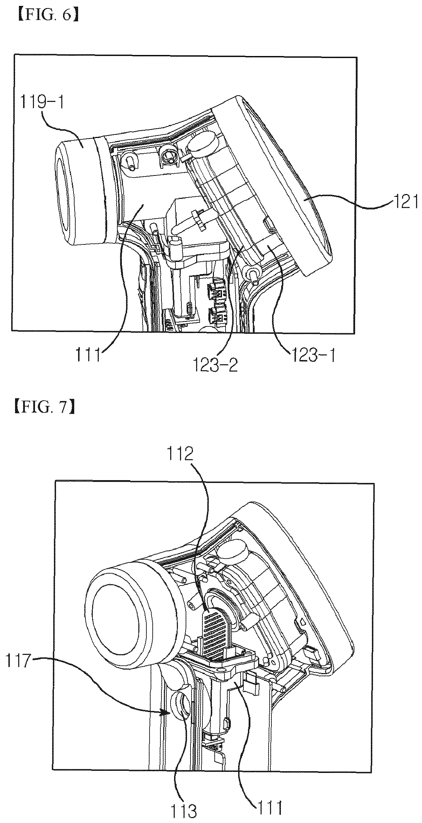

When referred along with FIG. 6, the first head part (221) of the housing may be disposedc with various elements for mist treatment, and the second head part (222) protruded from the other lateral surface may be disposed with other elements for temperature stimulation treatment.

A distal end surface of the first head part (221) may be formed with a raw water inlet to allow the water to be poured into a water tank (111) of the mist treatment part (110), and a distal end surface3 of the second head part (222) may be disposed with a skin contact member (121) applying a temperature stimulation.

That is, the first head part (221) may be related to raw water supply for generation of mist and water tank space arrangement, and the second head part (222) may be a portion that applies stimulation by being contacted to a user skin.

Meantime, when the first head part (221) is disposed at the same height as that of the second head part (222) or disposed at a height higher than that of the first head part (221), there may be generated a discomfort in the course of using the second head part (222).

For example, the process of a user stimulating a facial skin using the second head part (222) may be disturbed because of the first head part (221) being hitched at the bridge of a nose.

In relation thereto, as shown in FIG. 3, an uppermost end of the second head part (222) may be disposed at a height higher than that of the first head part (221) at a predetermined length (h).

Then, the discomfort that may be generated by the first head part (221) may be prevented when the temperature stimulation, the electroporation stimulation and light stimulation are applied to the skin through the distal end surface of the second head part (222). When the distal end surface of the second head part (222) is formed with a triangular shape as shown in the figure, there may be an advantage of the process being more convenient where a facial skin is cared including a nosal area using the second head part (222).

Furthermore, the size of the distal end surface of the second head part (222) is formed larger than that of the first head part (221), various functions implemented through the distal end surface of second head part (222) may become more conveniently useable. Although the given figures illustrate that the second head part (222) first starts to protrude from the handle part (210) ahead of the first head part (221) to thereby be formed with a broader size up to a position higher than the first head part (221), the present invention is not limited thereto.

Referring to FIGS. 6 and 7, the mist treatment part (110) may be formed by including a water tank (111) holding the raw water including the faucet water, an electrode module (112) disposed inside the water tank (111) to transform the raw water to sterilization water through electrolysis, an ultrasonic oscillator (113) generating a mist of fine particulate shape by applying vibration to the sterilization water generated by the electrode module (112), and a mist outlet (117) to allow the mist generated from the ultrasonic oscillator (113) to be ejected therethrough.

The structure, shape and material of water tank (111) may be variably organized as necessary, and the present invention is not particularly limited thereto.

A distal end surface of first head part (221) may be formed with a raw water inlet to allow the water to be poured into the water tank (111) of the mist treatment part (110), where the water tank (111) may be formed of an approximately '¬' shape, or a right-angled bend shape or a bent shape at approximately right-angle, across an upper space of the handle part (210) from the raw water inlet.

The raw water inlet formed at the distal end surface of first head part (221) may be opened/closed through an inlet cap (119-1), and a user may open the inlet cap (119-1), pour the raw water into the water tank (111), close the inlet cap (119-1) and tight-seal the raw water inlet.

Furthermore, an upper end of the handle part (210) may be disposed with a mist outlet (117) toward a direction where the raw water inlet is formed.

Each of the electrode modules (112) are formed to be spaced apart from the other, and may be formed by including a pair or more of electrode plates each connected to a negative pole (-) and a positive pole (+), and dissolve the water molecules using the principle of underwater discharge, whereby the sterilization water having the sterilizing power can be generated.

That is, the sterilizing power can be possessed by allowing various negative ions (O⁻, O₃⁻ , OH⁻, HOCl, H₂O₂) to be generated because plasma discharge is caused in the underwater through two electrode plates. The electrode modules (112) may be variably formed.

The ultrasonic oscillator (113) may be so disposed as to encompass a surrounding of the mist outlet (117) to efficiently spout the mist.

As one example, when the mist outlet (117) is formed to be round, the ultrasonic oscillator (113) may be formed with a ring shape to encompass a surrounding of the mist outlet (117).

With reference to mist generation using the ultrasonic oscillator (113), the shape of the water tank (111) at an area where the ultrasonic oscillator (113) is disposed may be formed with a front and rear length as narrow as possible and with a left and right length to be similar to an area of the ultrasonic oscillator (113).

Here, the front and rear length means a length between a surface where the ultrasonic oscillator (113) is disposed and an opposite surface thereto. Then, the mist ejection efficiency through the ultrasonic oscillator (113) can be enhanced.

For example, when the sterilization water is insufficient, a sufficient sterilization water can be supplied to the ultrasonic oscillator (113) for as long as possible. Because the sterilization water can be spread at an ultrasonic oscillator-disposed area as long as the water tank (111) is broad, an area can be reduced where the ultrasonic oscillator (113) and the sterilization water are contacted.

Furthermore, because the sterilization water contained at a position lower than the lowermost position of the ultrasonic oscillator (113) has a difficulty in being transformed to mist, the floor position of the water tank (111) may be so disposed as to be adjacent to the lowermost position of the ultrasonic oscillator (113).

As a result, an area contacted by the sterilization water and the ultrasonic oscillator (113) can be broadly maintained for as long as possible in the course of the sterilization water being reduced.

When a power is supplied to the electrode module (112) in response to the control of the controller (150), the electrolysis is performed to allow the raw water to be transformed to sterilization water, and the sterilization water mist is generated by the ultrasonic oscillator (113) to be spouted through the mist outlet (117).

FIG. 3 illustrates an example of a configuration where an ON/OFF switch for mist function is realized by an UP/DOWN sliding button (119-2) disposed underneath the mist outlet (117).

Although the electrode module (112) of mist treatment part (110) may be disposed at an arbitrary position inside the water tank (111), at least a portion of the electrode module (112) may be so disposed as to allow being looked through the raw water inlet, as an example related to visible effect.

Then, a user may visually ascertain an image of the sterilization water being generated by the electrode module (112) through the raw water inlet.

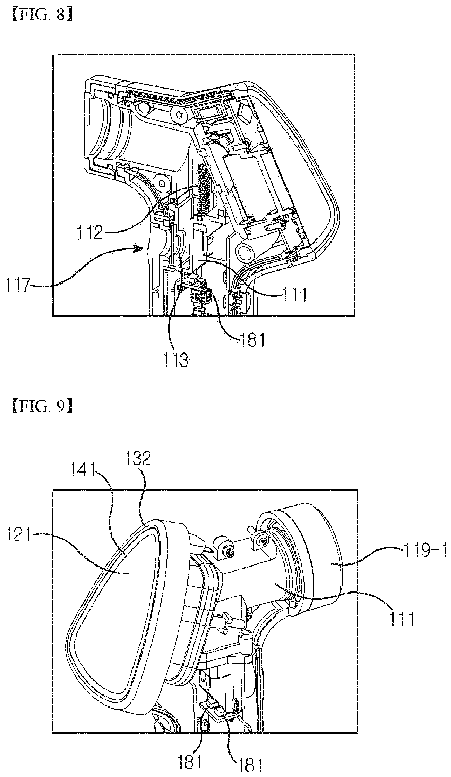

Referring to FIG. 8, the multi-functional integrated skin beauty device (100) may be formed by including an inlet cap (119-1) configured to open/close the raw water inlet, a visual effect plate (not shown) configured to display a visual effect by being so disposed at an inside of the water tank (111) as to allow being visible from the raw water inlet; and an illumination part (181) configured to shed an illumination light into an inside of the water tank (111) from under the floor of the water tank (111), wherein at least a portion of the water tank (111) is so disposed with a transparent or translucent material as to allow the illumination light coming off from the illumination part (181) to pass therethrough.

The visual effect seen from the raw water inlet may become more interesting due to the illumination light coming off through from the illumination part (181).

The illumination part (181) may be variably organized in order to shed the illumination light into the water tank (111). One of the examples is that the illumination part (181) may be formed using one or more L E Ds but the present invention is not limited thereto.

The visual effect plate (not shown) is a plated element disposed to provide a visual effect, and material, structure, size, shape and arrangement of the visual effect plate may be variably implemented.

For example, the visual effect plate may be displayed with various types of information and advertisements including characters, figures and images, and may be formed with material capable of demonstrating a special visual effect when a light is incident. However, the present invention is not limited thereto.

The visual effect plate may display a visual effect by being so disposed at an inside of the water tank (111) as to allow being visible from the raw water inlet.

The visual effect plate may be disposed at variable positions. As a detailed example, the visual effect plate may be disposed at an area where the water tank is curbed, at a front side (a side where the raw water inlet is situated) of electrode module (112), a rear side or an upper side of the electrode module (112). However, the present invention is not limited thereto.

When the visual effect plate is arranged, a user can see not only an image of the sterilization water being generated through the raw water inlet, and an inside image of the water tank seen in a subtle light by the illumination light but also various special effects including various types of information and holograms.

Furthermore, at least a portion of the inlet cap (119-1) may be so disposed as to allow an interior of the water tank (111) to look enlarged.

That is, the inlet cap (119-1) may be so disposed transparently or translucently as to allow a user to see an interior of the water tank (111) even when the raw water inlet is closed.

Furthermore, the inlet cap (119-1) may be so disposed as to allow an interior of the water tank (111) to look enlarged like a convex lens or reading glasses.

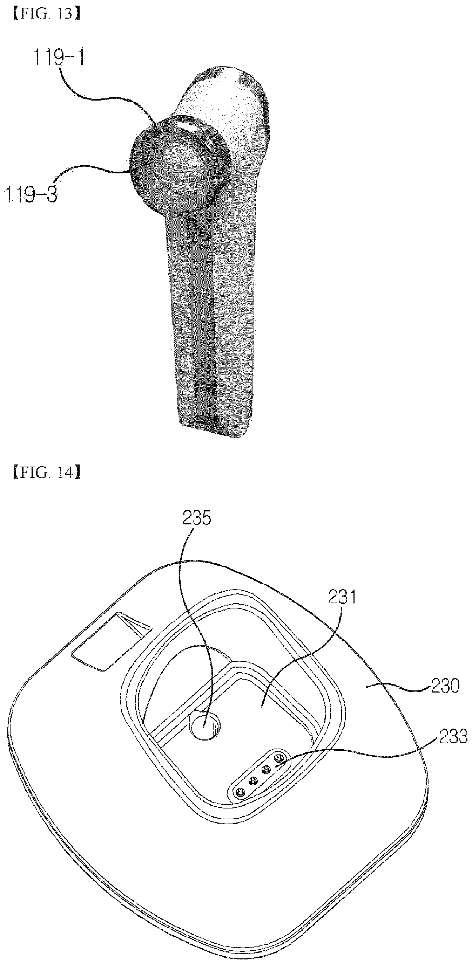

FIG. 13 illustrates a multi-functional integrated skin beauty device (100) according to a detailed exemplary embodiment of the present invention, and shows an example of an inner portion of the water tank (111) seen through a transparent reading glass material (119-3) while the inlet cap (119-1) is closed.

FIG. 9 illustrates the temperature stimulation function, the electroporation function and the light stimulation function being intensively provided on a distal end surface of the second head part (222) according to a detailed exemplary embodiment of the present invention.

The shape, structure and material of skin contact member (121) may be variably implemented.

For example, the skin contact member (121) may be formed with a material that enables an electric signal to be applied in order to have adequate heat transfer properties and to provide electroporation stimulation as well. Although the figure shows an example of skin contact member (121) formed with a triangular plated shape, the present invention is not limited thereto.

A transparent window (141) may be disposed along a surrounding of the skin contact member (121) in order to emit a light outputted from the light treatment part (140), and one or more of the LED elements forming the light treatment part (140) may be disposed at an inside of the transparent window (141). Furthermore, an outlying electrode member (132) may be disposed along an outer surrounding of the transparent window (141).

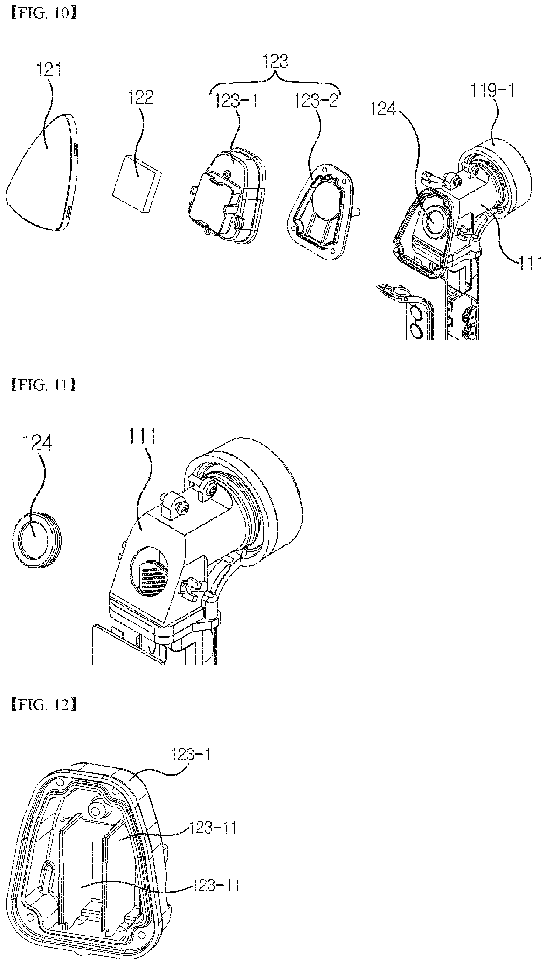

Referring to FIG. 10, the temperature stimulation treatment part (120) may be formed by including a skin contact member (121) applying a temperature stimulation by being contacted to a user skin, and a thermoelement (122) disposed to heat or cool the skin contact member (121).

The thermoelement (122) for temperature adjustment may be variably formed, and particularly formed using a peltier element.

The role of the skin contact member (121) may be so formed as to allow one surface of the peltier element (122) to directly perform.

One surface (external surface) of the peltier element (121) contacted by the skin contact member (121) may be treated with a particular temperature capable of stimulating the user skin. That is, when a current is applied to the peltier element (122) in response to the control of the controller (150), a relevant surface of the peltier element may be heated or cooled in response to the direction of the applied current.

Particularly, an inner surface of the peltier element (122) must be adequately cooled in order to perform a stable operation including protection of the peltier element.

Various methods for the said cooling may be utilized, and particularly, the inner surfaced of the peltier element (122) may be so disposed as to allow thermally contacting the water stored in the water tank (111) of the mist treatment part (110).

This means that the sterilization water stored in the water tank (111) of the mist treatment part (110) may be used for cooling of the peltier element (122).

The thermal contact between the inner surface of the peltier element (122) and the water stored in the water tank (111) of the mist treatment part (110) may be realized through a first heat dissipation part (124) of first material directly contacting the water stored in the water tank (111) and a second heat dissipation part (123) of second material disposed between the inner surface of the peltier element (122) and the first heat dissipation part (124).

That is, the water stored on the inner surface of the peltier element (122) and the water tank (111) may be thermally contacted by at least three mutually different elements.

Referring to FIG. 11, the first heat dissipation part (124) installed through a hole formed on the water tank (111) may contact, at one surface, the sterilization water inside of the water tank (111), and simultaneously contact the second heat dissipation part (123) at the other surface, where a portion installed with the first heat dissipation part (124) must be airtightly sealed by an O-ring to prevent the sterilization water from being leaked.

The first heat dissipation part (124) may be disposed at an external side (water tank side) of the second heat dissipation part (123) (see FIG. 10). The first heat dissipation part (124) may maximize the heat dissipation effect by being contacted with the sterilization water inside the water tank (111).

The shape of the first heat dissipation part (124) may not be particularly limited and may be variably formed such that the first heat dissipation part (124) may take a round shape, a polygonal shape and/or a star shape. When the first heat dissipation part (124) is disposed at an inner surface or at an outer surface with a plurality of lugs, the heat dissipation effect can be further enhanced.

Furthermore, a surface contacted by the first heat dissipation part (124) with the sterilization water of the water tank (111) may have the possibility of being contaminated by various ions and the like. Therefore, the first material may be formed with a material less contaminated by the sterilization water than the second material.

As a detailed example, the first material of first heat dissipation part (124) may be formed by including an SUS material and the second material of the second heat dissipation part (123) may be formed by including an aluminum material.

In this case, the surface contacted by the sterilization water of the water tank (111) may be minimized in oxidation.

As shown in FIG. 10, the thermal contact between the first heat dissipation part (124) and the second heat dissipation part (123) may be realized at an area where the water tank is curbed.

Meantime, the second heat dissipation part (123) may be formed by a water-cooling system that accommodates a cooling liquid therein in order to further improve the heat dissipation function. At this time, the second heat dissipation part (123) may be formed by including a main body (123-1) accommodating a cooling liquid and a lid (123-2).

Furthermore, as illustrated in FIG. 12, an inner space of main body (123-1) may be formed with a plurality of lugs (123-11) to further increase the cooling efficiency.

In the given figure, the lid (123-2) of the second heat dissipation part (123) may be so formed as to be fixed to the main body (123-1) using a bolt and a screw, and an O-ring may be disposed for airtight-sealing between the main body (123-1) and the lid (123-2).

The controller (150) may perform an overall control of the multi-functional integrated skin beauty device (100) in response to an operation command inputted from the input part (160).

The methods for controlling the multi-functional integrated skin beauty device (100) using the controller (150) may be variably implemented, but the present invention is not particularly limited thereto.

For example, the controller (150) may allow mist to be spouted by driving the mist treatment part (110) in response to ON/OFF signals of mist function inputted from the input part (160), and may maintain the skin contact member (121) to be under or at an adequate temperature by driving the temperature stimulation treatment part (120) in response to cold/warm stimulation set-up signals.

Furthermore, the controller (150) may controllably allow generating an electric stimulation through the electroporation electrode by driving the EP treatment part (130) in response to ON/OFF signals of the electroporation function, and shedding an adequate light to be irradiated through the transparent window (141) by driving the light treatment part (140) in response to ON/OFF signals of light stimulation function.

The housing may be disposed with various display means in order to display the operations of the multi-functional integrated skin beauty device (100). For example, the housing may be disposed with LED lamps for displaying various types of information, including but not limited to, power ON/OFF state, operation state of mist treatment part (110), operation state of temperature stimulation treatment part (120), operation state of EP treatment part (130), operation state of light treatment part (140), and current cooling/warming set-up state.

The power supply part (170) may be so configured as to transform a DC power to an AC power for use in the multi-functional integrated skin beauty device (100), and may be so configured as to include a chargeable battery. The power supply part (170) may be variably configured to include a battery charging circuit as necessary.

Various other components including PCBs and batteries forming an electric circuit may be disposed inside an inner space of handle part (210).

The multi-functional integrated skin beauty device (100) may include a cradle part (230) performing a role of a resting stand or a mount.

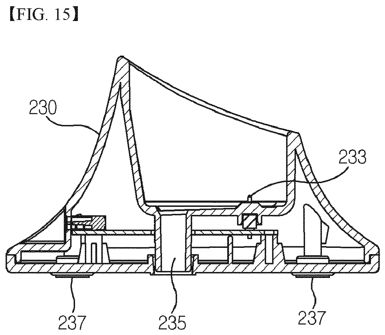

Referring to FIGS. 14 and 15, the cradle part (230) may be so configured as to allow a lower end of the handle part (210) to rest thereon, and the cradle part may be so disposed as to function as a battery for charging. At this time, a battery charging terminal (233) may be formed on the handle part (210) and the cradle part (230) for a mutual electric contact when the handle part (210) is mounted.

FIG. 14 illustrates a cradle part (230) viewed from above according to an exemplary embodiment, where a floor (231) of resting space of the cradle part (230) may be formed with a water drain hole (235) to allow the water flowing down through the handle part (210) to be drained out.

That is, the mists formed as water droplets in the course of being continuously spread may flow down along each portion of the handle part (210) including the mist function ON/OFF switch (119-2).

Thus, when the floor of the cradle part (230) is formed with a water drainage hole (235), water leaked through the handle part (210) may be drained out, whereby safety can be enhanced by preventing a short-ci rcuit from bei ng generated on the battery charging terminal (233) .

Referring to FIG. 15, a portion formed with the water drain hole (235) at the cradle part (230) may be formed to be lower than a portion disposed with the battery charging terminal (233).

At this time, a descent slope may be formed at a predetermined angle from a portion where the battery charging terminal (233) is disposed to a portion where the water drainage hole (235) is formed.

Furthermore, a lower surface of cradle part (230) may be formed with a prop (237) and a water vessel (not shown) may be disposed to contain water drained through the water drainage hole (235).

While the present invention has been described with reference to the particular illustrative embodiments, it is not to be restricted by the embodiments but only by the appended claims. It is to be appreciated that those skilled in the art can change or modify the embodiments without departing from the scope and spirit of the present invention.

### [DESCRIPTION OF REFERENCE NUMERALS]

| | |
|---|---|
| 100: beauty device | 110: mist treatment part |
| 111: water tank | 112: electrode module |
| 113: ultrasonic oscillator | 117: mist outlet |
| 119-1: inlet cap | 120: temperature stimulation treatment part |
| 121: skin contact member | 122: thermoelement (peltier device) |
| 123: second heat dissipation part | 123-1: main body |
| 123-2: lid | 124: first heat dissipation part |
| 130: EP treatment part | 132: outlying electrode member |
| 140: optical treatment part | 141: transparent window |
| 150: controller | 160: input part |
| 170: power supply part | 181: illumination part |
| 210: handle part | 220: head part |
| 221: first head part | 222: second head part |
| 230: cradle part | 233: battery charging terminal |
| 235: water drainage hole | 237: prop |

## Claims

1. A multi-functional integrated skin beauty device, the device comprising:
a mist treatment part generating sterilization water by electrolyzing water in a water tank and spraying the generated sterilization water in a mist shape;
a temperature stimulation treatment part applying a temperature stimulation to skin using an thermoelement;
a power supply part supplying an electric power;
an input part receiving an operation command; and
a controller controlling the mist treatment part and the temperature stimulation treatment part in response to the operation command inputted through the input part, wherein a housing forming an exterior appearance of the multi-functional integrated skin beauty device includes:
a pillar-type handle part graspable by a user with one hand; and
a first head part protruded from an upper end of the handle part toward a lateral surface, and a second protruded toward an opposite lateral surface thereof, and wherein the first head part is disposed with a raw water inlet to allow raw water to be poured into a water tank of the mist treatment part and the second head part is disposed with a skin contact member to allow applying a temperature stimulation by being contacted with skin.

2. The device of claim 1, further comprising:
an E P treatment part applying an electric signal to an E P electrode; and
a light treatment part outputting a light for skin care, wherein
the second head part is disposed with a transparent window formed along a surrounding of the skin contact member to output a skin care light, and wherein
the skin contact member and an outlying electrode member formed at a surrounding of the transparent window form the E P electrode, and wherein an uppermost end of the second head part is disposed higher than the first head part.

3. The device of claim 1, wherein the mist treatment part includes:
an electrode module disposed inside of the water tank to transform the raw water to sterilization water by electrolyzing the raw water;
an ultrasonic oscillator generating a fine particulate type mist by applying a vibration to the sterilization water generated by the electrode module; and
a mist outlet to allow spouting the mist generated by the ultrasonic oscillator therethrough, wherein
the water tank is formed of an approximately '¬' shape across an upper space of the handle part from the raw water inlet, and wherein
at least a portion of the electrode module is so disposed at a position as to be visible through the raw water inlet.

4. The device of claim 3, wherein the mist outlet is disposed at an upper end of the handle part toward a direction formed with the raw water inlet, and the ultrasonic oscillator is disposed at a surrounding of the mist outlet, wherein a floor position of the water tank is so disposed as to be adjacent to a lowermost position of the ultrasonic oscillator.

5. The device of claim 1, further comprising:
an inlet cap configured to open/close the raw water inlet;
a visual effect plate configured to display a visual effect by being so disposed at an inside of the water tank as to allow being visible from the raw water inlet; and
an illumination part configured to shed an illumination light into an inside of the water tank from under the floor of the water tank, wherein
at least a portion of the water tank is so disposed with a transparent or translucent material as to allow the illumination light coming off from the illumination part to pass therethrough, and wherein
at least a portion of the inlet cap is so disposed as to allow an interior of the water tank to look enlarged.

6. The device of claim 1, further comprising: a cradle part so disposed with a battery charging terminal as to allow a lower end of the handle part to rest thereon, wherein
the cradle part is disposed at a floor with a water drain hole to allow the water flowing down through the handle part to be drained out, and wherein a portion formed with the water drain hole is formed to be lower than a portion disposed with the battery charging terminal.
